# EUROPEAN PATENT SPECIFICATION

(11) **EP 3 122 306 B1**
(45) Date of publication and mention of the grant of the patent: **17.11.2021**
(21) Application number: 15768231.1
(22) Date of filing: 25.03.2015
(51) Int. Cl.: A61H 7/00, H05F 1/00, A61N 1/00, H02N 1/00, A61N 1/10, H02N 1/04

(54) **STATIC ELECTRICITY GENERATOR**
STATISCHER ELEKTRIZITÄTSGENERATOR
GÉNÉRATEUR D'ÉLECTRICITÉ STATIQUE

(30) Priority: 25.03.2014 US 201461969841 P
(43) Date of publication of application: 01.02.2017
(73) Proprietor: Eliachar Technologies Development Ltd, 34371 Haifa (IL); HAR-SHAI, Gadi, Hod HaSharon (IL)
(72) Inventor: HIRSHOWITZ, Bernard, Haifa (IL); HIRSHOWITZ, Bernard, 34482 Haifa (IL); LILACH, Nir, 3658200 Kfar Yehoshua (IL); ELIACHAR, Eliahu, 34371 Haifa (IL)
(74) Representative: Lecomte & Partners
(86) International application number: PCT/IL2015/050310
(87) International publication number: WO 2015/145436

(56) References cited:
- WO-A1-2007/050144
- WO-A2-2010/132536
- GB-A- 159 358
- RU-C1- 2 473 371
- US-A- 2 644 903
- US-A- 4 915 110
- US-A- 5 506 491
- US-A1- 2009 140 603
- US-A9- 2007 192 972

## Description

### FIELD OF THE INVENTION

The present invention generally pertains to a static electricity generator. More specifically, the invention relates to a portable handheld static-electricity device useful for decreasing and alleviating pain of a patient by means of a static electricity generator comprising a polymer sheet assembly and a brush assembly.

### BACKGROUND OF THE INVENTION

Myalgia (muscle pain) and joint paint are each a common symptom of many diseases and disorders. Many treatments were suggested art to alleviate the pain, including application of heat and electricity upon the sour organ, and using static electricity, for example :
US patent No. 4915110 provides a non-invasive body contacting device for effecting electrostatic therapy to selected points of the body. The device comprises a first electrical current conductive pin-like element (8) and at least one further current conductive member disposed in proximity to the element. At least the body facing surfaces of the element and the member are provided with current insulating material. The element and the member are provided with means connectable to a low power source.
US patent App. No. 2013296748 relates to the massage methods and electric massage devices for applying the friction with pressure to the body surface with the object of improvement of body condition as well as diagnosis, prevention and treatment diseases. Additional possibilities presented in US2013296748 are that the friction between the skin of patient and the end of influencing head of the massage device creates the electrostatic potential on the body surfaces or brings the useful substances such as medicine or the anti-inflammatory metal such cooper or silver to the skin's surface. Moreover, the electrostatic potential on the body surfaces has the anti-inflammatory and the pain relief effects.
PCT App. No. 2006060304 discloses a portable Static Electro Magnetic Field (SEMF) generating device and method for the therapeutic treatment of inflammatory and painful disorders of the human limbs including fingers, hands, wrists, forearms, elbows, toes, feet and ankles preferably includes a wearable, portable battery powered solenoid or coil. The coil is configured to focus a substantially time invariant magnetic flux field onto a selected part of the body. The SEMF generating device's coil, when energized, works by slightly changing the charges associated with voltage-dependent ion channels in the cell membranes of the human body, thereby stabilizing abnormal fluid transport into the cells through aquaporin channels and the sodium ions relating to them, treating inflammation and altering the flow of calcium, chloride and potassium ions through their respective neuronal ion channels and treating pain.

Another device for generating electrostatic charge is disclosed by US2007/192972A1.

Various methods devices for discharging static electricity were suggested yet none of them introduces a static electricity generator which is effectively alleviating pain.

It therefore remains a long felt and unmet need to provide novel means and methods for a static electricity generator which is effectively alleviating pain without using electrodes or clamps.

### SUMMARY OF THE INVENTION

It is hence an object of the invention to provide a portable handheld static-electricity generator for alleviating pain in at least one portion of patient's body, comprising
a. a movable brush assembly,
b. an automatically-operated mechanical unit;
wherein additionally comprising a polymer assembly comprising a rubbing-surface portion upon which the brush rubs; the polymer assembly having affinity to a defined charge such that when the movable brush rubs the rubbing surface of the polymer assembly for a predefined time period a static electricity is generated .

Preferred embodiments are defined by the dependent claims.

### BRIEF DESCRIPTION OF THE DRAWINGS

In order to understand the invention and to see how it may be implemented in practice, a few preferred embodiments will now be described, by way of non-limiting example only, with reference to be accompanying drawings, in which:
**Fig. 1** illustrates a top view of the static electricity generator device of the present invention;
**Fig. 2** illustrates a top and bottom view of the static electricity generator device of the present invention;
**Fig. 3** illustrates a top and bottom view and bottom view of the mechanical unit and the polymer sheet assembly, of the present invention;
**Fig. 4** illustrates a top view of the polymer sheet assembly of the present invention;
**Fig. 5** illustrates a top view of the mechanical units of the static electricity generator device of the present invention;
**Fig. 6** illustrates a side view of the mechanical units of the static electricity generator device of the present invention;
**Fig. 7** illustrates a top view of the static electricity generator device of the present invention;
**Figs. 8-9** illustrate a side view of the static electricity generator device of the present invention;
**Figs. 10-14** illustrate graphs of daily operation of the portable handheld static-electricity generator presenting the electrostatic field vs. time period, of the present invention; and,
**Figs. 15-18** illustrate the variety of the brush motions configuration of the present invention.

### DETAILED DESCRIPTION OF THE INVENTION

A portable handheld static-electricity generator for alleviating pain in at least one body portion of a patient is hereby presented in a non-limiting manner. This electro-static field generating medical device is characterized by a movable brush , a polymer assembly which comprising a rubbing-surface portion upon which the brush rubs such that static electricity is generated . The polymer sheet assembly further comprising a discharge portion which is in contact with at least one portion of patient's body

The term *"**polymer sheet assembly***" broadly refers hereinafter, in a non-limiting manner to any material which is with a negative charge or positive thereby, is with stronger affinity for negative charge such as silicone rubber, silicon, Teflon, PVC, polyethylene and rubber. Furthermore the term polymer refers to a sheet, beads or pieces of rubber, plastic, glass, pith, and polymer in any form which is made from inert, non-reactive material, for example polydimethylsiloxane or related polysiloxane materials (represented generally by the structure R₂-Si-O, where R is a monovalent organic radical, including for example a methyl, ethyl, propyl or phenol group, among others and which may be several thousands of S-O units or more in length and that can build up static electricity on it.

In another embodiment of the present invention the polymer sheet assembly may be a semi conductive material such silicon.

The term "***brush assembly**"* broadly refers hereinafter, in a non-limiting manner to any material which is with a positive charge thereby, is with stronger affinity for positive charge. The brush is composed of a material selected from the group consisting of Polyurethane foam, hair, nylon, Nylatron, glass, paper cotton and any combination thereof.

As used herein the term *"****about* X***"* or *"****approximately* X**" refers to a range 25% less than to 25% more than of X (X ± 25%), at times X ± 20%, X ± 15%, X ± 10% and preferably X ± 5%.

According to one embodiment of the invention, the device is characterized by an automatically-operated mechanical unit, a brush assembly and a polymer sheet assembly. The polymer sheet assembly comprising a first rubbing-surface portion upon which the static electricity is generated and a second surface portion attached to the patient's body, through which the charge is discharged. The first rubbing-surface portion is the upper surface and the second surface portion is the lowest portion of the assembly, opposite to the first portion.

The mechanical unit comprising a DC motor and gear-head thereof, provided in connection with a spur gear by means of a worm gear. The brush assembly comprises a brushing member made of at least one material selected from the group consisting of Polyurethane foam, hair, nylon, Nylatron, glass, paper, cotton, Kevlar, rubber, fabric, leather or any other material having affinity to positive charges. The brush further comprises a brushing material which is in contact with the polymer sheet assembly and a brush body such as a handle or a block which the brushing material is affixed to either parallel- or perpendicular-wise. The brush body is further reversibly or irreversibly attached on the spur gear. The polymer sheet assembly comprises a polymer sheet and base thereof. This sheet is shape in a suitable manner: a planer sheet or 3D designed one (e.g., with a concaved or polygonal structure etc.), smooth or with textured surface etc. This polymer sheet is positioned adjacent to the brushing member, pressed against the polymer sheet inner surface by a spring such that by operating the motor, the spur gear activates or actuates the brush, which is continuously rubbing the polymer sheet for a predefined time period t thereby, generating static electricity upon the sheet, by attaching the electrically-loaded polymer sheet to a patient's body portion, pain is alleviated. The attachment force of the brush to the polymer sheet, which determines the contact surface area and the resultant electrostatic field, can be adjusted by selecting a preferred spring. Furthermore, the effect is greatly enhanced by rubbing the materials, the brush and the polymer sheet assembly together, as they touch and separate many times. For surfaces with differing geometry, rubbing may also lead to heating of protrusions, causing pyroelectric charge separation which may add to the existing contact electrification, or which may oppose the existing polarity. The polarity and strength of the charges produced differ according to the materials, surface roughness, temperature, strain, and other properties.

In another embodiment of the present invention, the static-electricity generator device provides therapeutic treatment of inflammatory and painful disorders and is configured to be held against or supported upon human limbs including fingers, hands, wrists, forearms, elbows, toes, feet or ankles the device is a wearable device having configuration of a belt, a bracelet, a strap, a strip and the like.

In another embodiment of the present invention, the brush may be provided with an Activated Configuration in which moving brush is rubbed against the sheet so as static electricity is generated and a Non-Activated Configuration in which the brush is detached from the sheet and static electricity is not generated and further not discharged due to the detachment.

Without wishing to be bound by theory, when two different materials are pressed or rubbed together, the surface of one material will generally 'steal' some electrons from the surface of the other material. The material that steals electrons has the stronger affinity for negative charge of the two materials, and that surface will be negatively charged after the materials are separated. Thereby, when material come into frictive contact, a contact electrification occurs, in which certain materials become electrically charged. The surface of the material is electrically charged, either negatively or positively, any contact with an uncharged conductive object or with an object having substantially different charge may cause an electrical discharge of the built-up static electricity.

The polarity and strength of the charges produced differ according to the materials, surface roughness, temperature, strain, and other properties.

If various insulating materials are pressed or rubbed together and then the amount and polarity of the charge on each surface is separately measured, a very reproducible pattern emerges.

Table 1 below is a Triboelectricity table which presents materials having positive affinity v.s. negative affinity and how strong the effect will be:

**Table 1**

| **Material** | **Affinity nC/J** | **Characteristics** |
|---|---|---|
| Polyurethane foam | +60 | Good insulators (>1000 T ohm cm) |
| Sorbothane | +58 | Slightly conductive. (120 G ohm cm). |
| Box sealing tape (BOPP) | +55 | Non-sticky side. Becomes more negative if sanded down to the BOPP film. |
| Hair, oily skin | +45 | Skin is conductive. Cannot be charged by metal rubbing. |
| Solid polyurethane, filled | +40 | Slightly conductive. (8 T ohm cm). |
| Magnesium fluoride (MgF2) | +35 | Anti-reflective optical coating. |
| Nylon, dry skin | +30 | Skin is conductive. Cannot be charged by metal rubbing. |
| Machine oil | +29 | |
| Nylatron (nylon filled with MOS₂) | +28 | |
| Glass (soda) | +25 | Slightly conductive. (Depends on humidity). |
| Paper (uncoated copy) | +10 | Most papers & cardboard have similar affinity. Slightly conductive. |
| Wood (pine) | +7 | |
| GE brand Silicone II (hardens in air) | +6 | More positive than the other silicone chemistry (see below). |
| Cotton | +5 | Slightly conductive. (Depends on humidity). |
| Nitrile rubber | +3 | |
| Wool | 0 | |
| Polycarbonate | -5 | |
| ABS | -5 | |
| Acrylic (polymethyl methacrylate) and adhesive side of clear carton-sealing and office tape | -10 | Several clear tape adhesives are having an affinity almost identical to acrylic. |
| Epoxy (circuit board) | -32 | |
| Styrene-butadiene rubber (SBR, Buna S) | -35 | Sometimes inaccurately called "neoprene" (see below). |
| Solvent-based spray paints | -38 | May vary. |
| PET (Mylar) cloth | -40 | |
| PET (Mylar) solid | -40 | |
| EVA rubber for gaskets, filled | -55 | Slightly conductive. (10 T ohm cm). Filled rubber will usually conduct. |
| Gum rubber | -60 | Barely conductive. (500 T ohm cm). |
| Hot melt glue | -62 | |
| Polystyrene | -70 | |
| Polyimide | -70 | |
| Silicones (air harden & thermoset, but not GE) | -72 | |
| Vinyl: flexible (clear tubing) | -75 | |
| Carton-sealing tape (BOPP), sanded down | -85 | Raw surface is very + (see above), but close to PP when sanded. |
| Olefins (alkenes): LDPE, HDPE, PP | -90 | UHMWPE is below. Against metals, PP is more negative than PE. |
| Cellulose nitrate | -93 | |
| Office tape backing (vinyl copolymer | -95 | |
| UHMWPE | -95 | |
| Neoprene (polychloroprene, not SBR) | -98 | Slightly conductive if filled (1.5 T ohm cm). |
| PVC (rigid vinyl) | -100 | |
| Latex (natural) rubber | -105 | |
| Viton, filled | -117 | Slightly conductive. (40 T ohm cm). |
| Epichlorohydrin rubber, filled | -118 | Slightly conductive. (250 G ohm cm). |
| Santoprene rubber | -120 | |
| Hypalon rubber, filled | -130 | Slightly conductive. (30 T ohm cm). |
| Butyl rubber, filled | -135 | Conductive. (900 M ohm cm). Test was done fast. |
| EDPM rubber, filled | -140 | Slightly conductive. (40 T ohm cm). |
| Teflon | -190 | Surface is fluorine atoms- very electronegative. |

The materials mentioned in Table 1 above having negative affinity can be used as a polymer sheet assembly whilst the material having positive affinity may further be used for the brush assembly.

In another embodiment of the present invention, the materials mentioned in Table 1 above having negative affinity can be used as a brush assembly polymer sheet assembly whilst the material having positive affinity may further be used for the polymer sheet assembly.In another embodiment of the present invention, the mechanical device, together with the battery pack is placed within a specialized fabric structure, which attaches the mechanical unit and the battery pack to the patient's body.

In another embodiment of the present invention, the generator device of the present invention may be operated by at least two external batteries of about 1.5 [V] and about 3[V]. The battery may be a rechargeable battery.

In another embodiment of the present invention, the device comprising the polymer assembly discharges slowly a static electricity over time covering a large body surface.

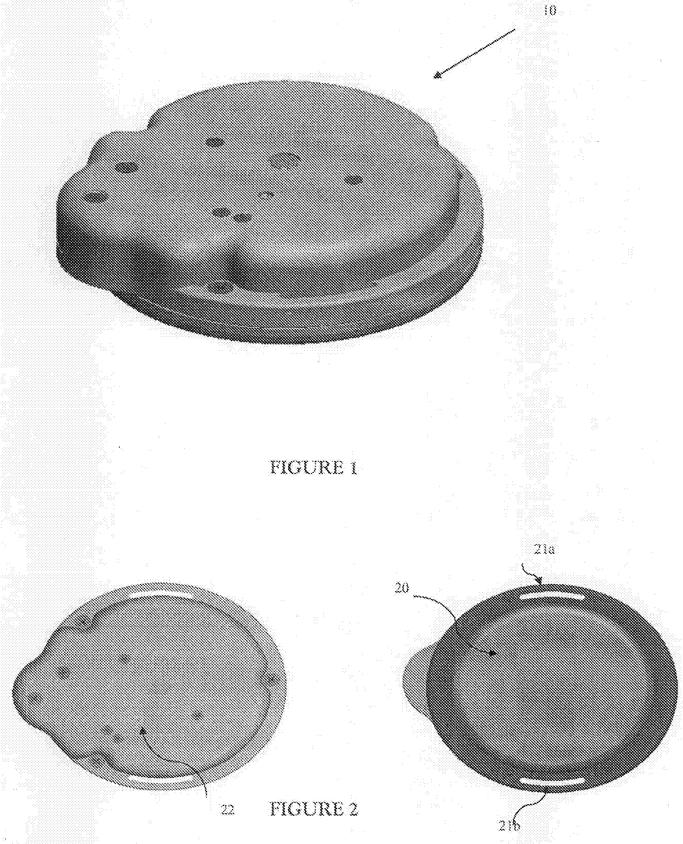

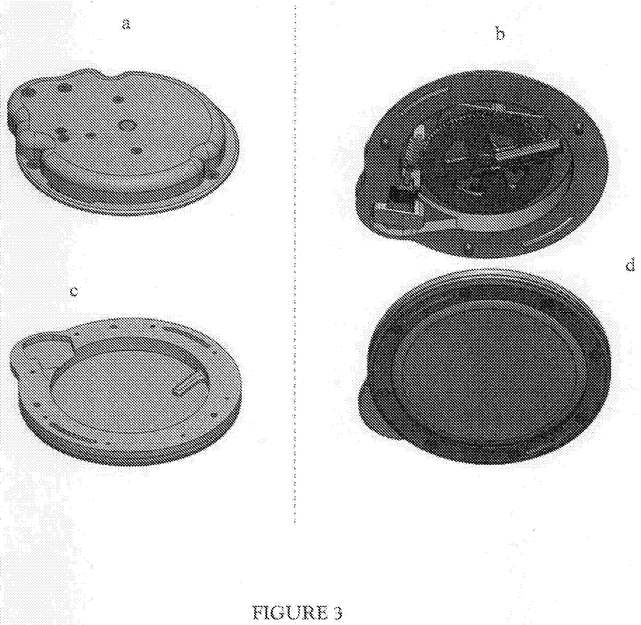

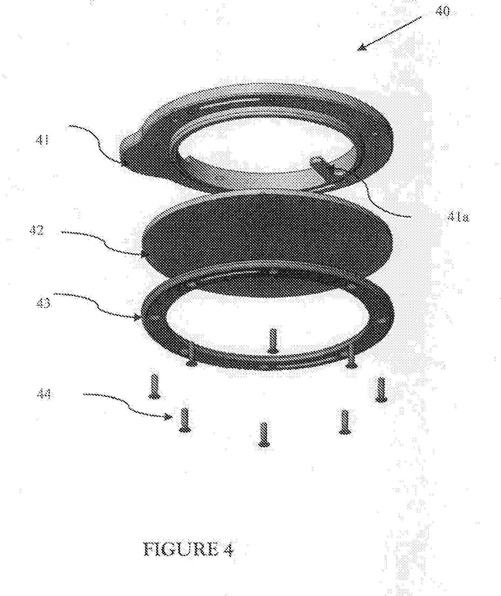

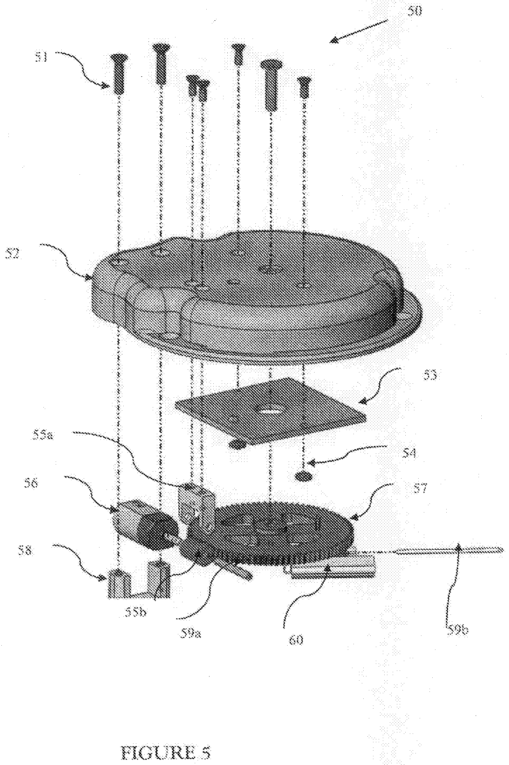

An embodiment of the invention is hereby schematically illustrated in a non-limiting manner:
Reference is now made to **Figures 1-2** which illustrate a top view and bottom view of the electrostatic generator comprising a polymer sheet assembly such as silicone 10 and at least one slot for a strip 11a-b. The device may further comprise a LED unit 12.
Reference is now made to **Figure 3** which illustrates a top view and bottom view of the electrostatic generator comprising a polymer sheet assembly. Figures 3a-b illustrate a top and bottom view of the mechanical unit and figs. 3c-d illustrate a top and bottom view of the polymer sheet assembly.
Reference is now made to **Figure 4** which illustrates the polymer sheet assembly 40 comprising a base with a brush lifter unit 41a, a polymer sheet 42 such as silicone, an attachment washer 43 such as Acetal and plurality of stainless steel screws 44.
The brush lifter unit is configured to hold the brush such that the brush is detached from the sheet and static electricity is not discharged thereby, providing the brush in a Non-Activated Configuration.
Reference is now made to **Figure 5** which illustrates the mechanical unit 50 of the electrostatic generator of the present invention.

The mechanical unit comprises a plurality of stainless steel screws 51, a cover 52 composed of Acetal PCB 53, an Acetal worm gear holder, a stainless steel nut 54, a worm gear 55a-b, an DC motor and gearhead 56, a spur gear (e.g. reduction gear) having 90 teeth 57, an Acetal, motor holder 58, a stainless steel shaft 59a-b and a brush 60.

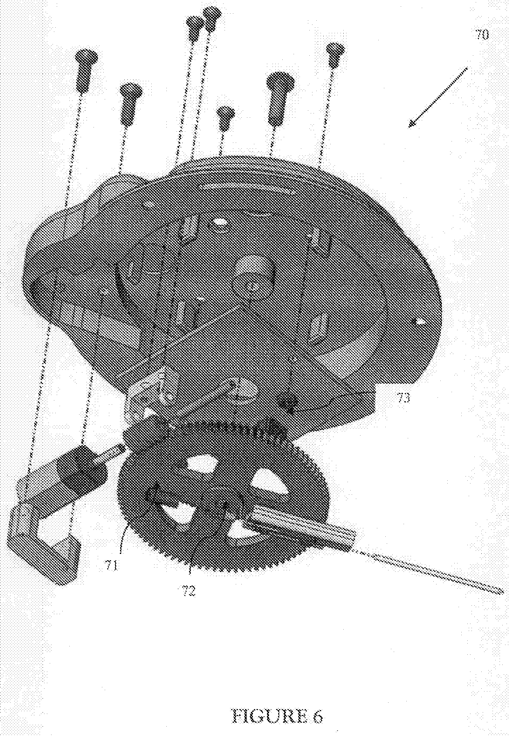

**Figure 6** further illustrates a side view of the electrostatic generator 70 comprising a stainless steel spring 72 an Acetal stopper 71 and a photomicrosensor 73.

The electro static generator of the present invention further comprises a predetermined protocol of treatment comprising a range of electrical charges from about 2.5 to about 15 kilo Volts per inch. The protocol includes a plurality of predetermined sequences or sessions of charges for obtaining electrostatic field and durations of time specific to treat a surface of interest and further reducing pain.The predetermined protocol includes 6 to 10 sessions, preferably 8 sessions, whilst the first sequence of charges may increase linearly and continuously from a first charge to a second, greater, charge and a second sequence of charges decreasing linearly and continuously from the second charge to the first charge.

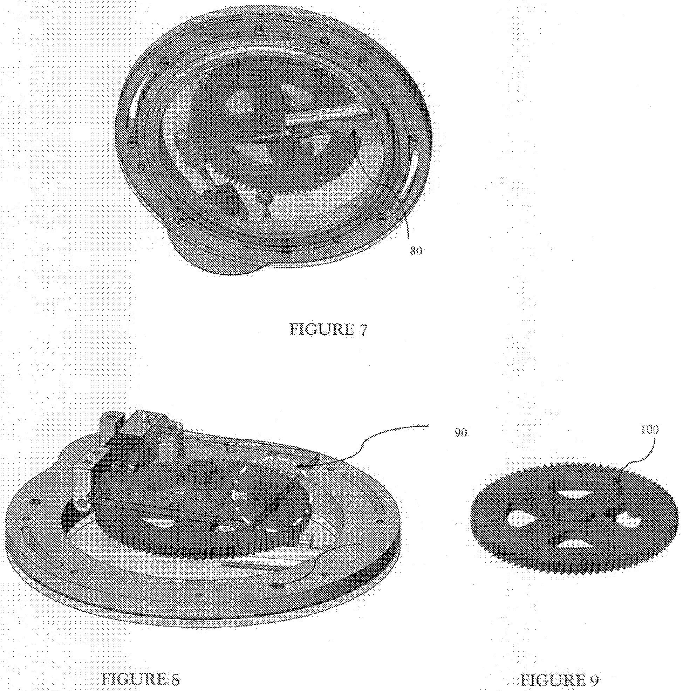

Reference is now made to **Figure 7** which illustrates a brush lifting element 80 and an element which detaches the brush from the polymer sheet assembly.

According to an embodiment of the invention, each session lasts about t seconds; t is ranging, for example, from about 15 to about 20 minutes; whereas t can be modified by the patient or by the physician. By the end of the session, the brush is detached from the polymer sheet. This is achieved by the following procedure: after predefined duration or otherwise by means of an online-feedback manner lasting t seconds, the brush meets a brush lifter as illustrated in figure 7, which detaches it from the polymer sheet. At the same time, a 'fin' from the spur gear get into the photo-microsensor which notify the software that the brush has been detached and the motor is stopped.

**Figure 8** further illustrates the point where the brush meets the lifting element and detaches from the polymer assembly. The bolt element of the spur ('fin') gets into the photomicrosensor. **Fig. 9** further presents the 'fin' rises up from the spur.

In another embodiment of the present invention , a system for alleviating pain in at least one portion of patient's body comprising a portable handheld static-electricity generator for alleviating pain in at least one portion of patient's body and a central processing unit (CPU) for controlling and activating the generator according to a predetermined protocol.

### EXAMPLE 1

Without forming part of the invention, a method for alleviating pain in patient's body or a portion thereof is presented: The method comprises, *inter alia,* steps of (*a*) providing a portable handheld static-electricity generator as defined in any of the above, which comprises, inter *alia,* a movable brush and polymer sheet assembly comprising a rubbing-surface upon which the brush rubs;(b) actuating the brush in respect to the sheet thereby rubbing the sheet by means of the brush; (c) generating a static electricity (electro-static field); and (d) attaching the sheet to patient's body so that pain is alleviated.

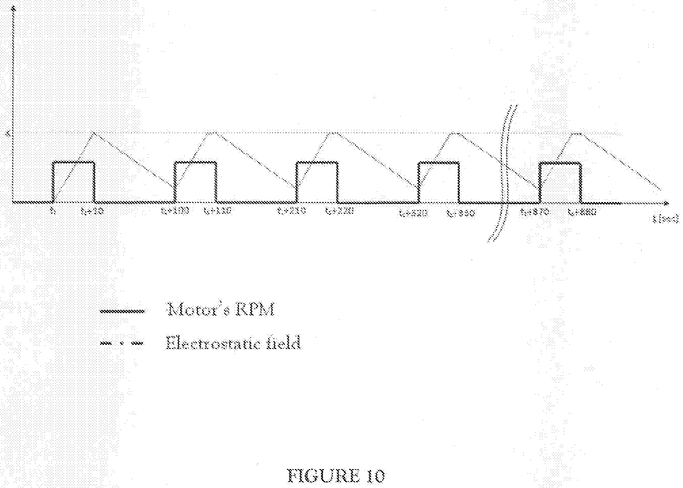

Reference is now made to Figure 10 which illustrates a graph of the electrostatic field generated from the electrostatic generator device of the present invention vs a time period (t). The charging time of the generator is 10 seconds.

Daily operation of the portable handheld static-electricity generator as defined in the aforesaid embodiment in 6 to 10 sessions per day, of about 15 to about 20 minutes each, alleviates joint pain and improves Wong-Backer Face Scale in one unit after one week.

### EXAMPLE 2

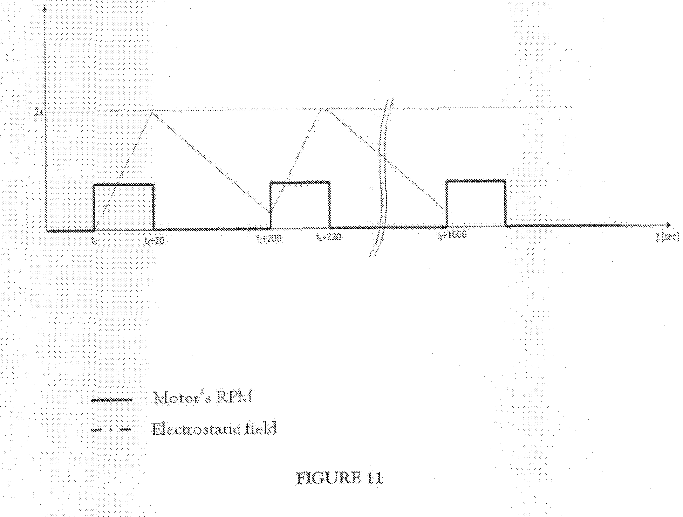

Reference is now made to Figure 11 which illustrates a graph of the electrostatic field generated from the electrostatic generator device of the present invention vs time period. The charging time of the generator is 20 seconds.

Daily operation of the portable handheld static-electricity generator as defined and described in the aforesaid embodiment in 6 to 10 sessions per day, of about 15 to about 20 minutes each, (a continuous electrostatic field), alleviates muscle pain and improves Wong-Backer Face Scale in one unit after one week of treatment.

### EXAMPLE 3

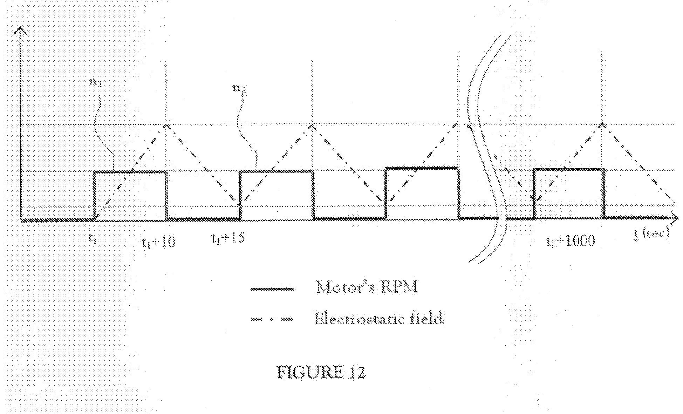

Reference is now made to Figure 12 which illustrates a graph of the electrostatic field generated from the electrostatic generator device of the present invention vs a time period (t). the graph illustrated the motor's RPM (frequency of rotations around a fixed axis in one minute) which is further proportional to the brush's RPM.

Daily operation of the portable handheld static-electricity generator as defined and described in aforesaid embodiment in 6 to 10 sessions per day, of about 15 to 20 minutes each (each session comprises introduction of a pulsed electrostatic field, *n*=*10*), alleviates muscle pain and improves Wong-Backer Face Scale in one unit after one week of treatment:

### EXAMPLE 4

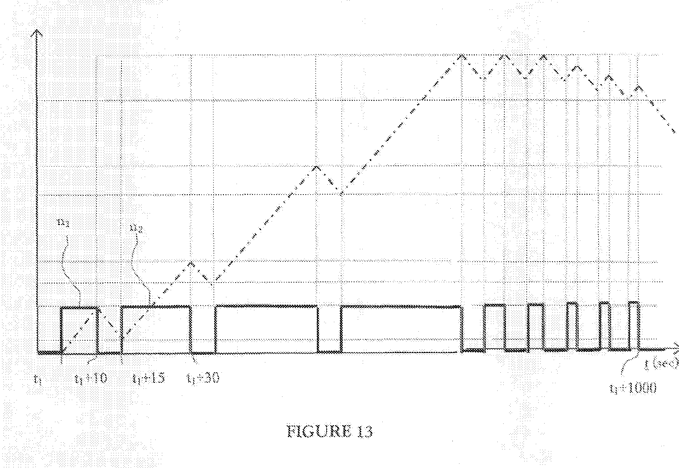

Reference is now made to Figure 13 which illustrates a graph of the electrostatic field generated from the electrostatic generator device of the present invention vs a time period (t).

Daily operation of the portable handheld static-electricity generator as defined and described in aforesaid embodiment and further comprising electrical means for regulating electrostatic field (coil, capacitor etc.) in 6 to 10 sessions per day, each treatment provides for about 15 to about 20 minutes (at least half of the sessions include introduction of a pulsed electrostatic field, n=9), alleviates joint pain and improves Wong-Backer Face Scale in one unit after one week of treatment:

### EXAMPLE 5

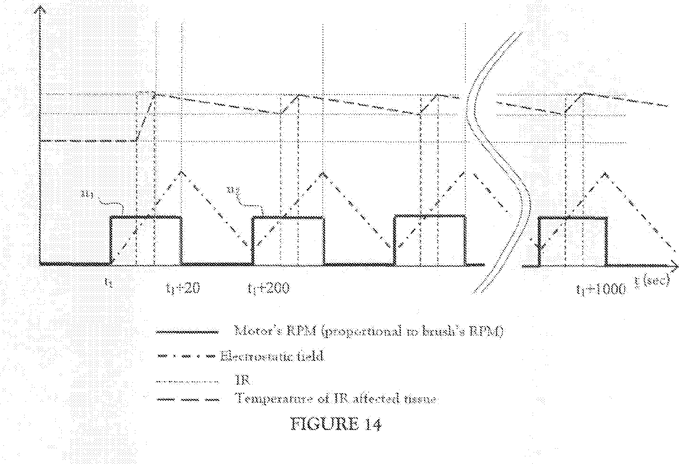

Reference is now made to Figure 14 which illustrates a graph of the electrostatic field generated from the electrostatic generator device of the present invention vs a time period (t).

Daily operation of the portable handheld static-electricity generator as defined and described above, which further comprises an infrared light (IR) emitter, and further comprising electrical means for regulating electrostatic field (coil, capacitor etc.) in 6 to 10 sessions sessions per day, each treatment extends between 15 to 20 minutes (at least half of the sessions include introduction of a pulsed electrostatic field, n=9), alleviates joint pain and improves Wong-Backer Face Scale in one unit after one week of treatment. The graph further illustrates the temperature of IR affected tissue.

### EXAMPLE 6

Another object of the present disclosure is to provide means and methods of a non-invasive treatment for chronic pain relief by utilizing a sheet of occlusive polymer of a thickness between about 2 and 4 mm thickness.

Hence according to an embodiment of the invention, an attached mechanism generates in either a continuous or pulse-wise manner a negative static electrical charge of about 2.5 to about 15 Kilo Volts per inch that is applied to the polymer, providing large amount of negative ions that are being used for treatment.

The continuous charge of ions avoids electrical charge decay due to environmental and patient conditions such as humidity, temperature and perspiration. The negative ions on the polymer repel circulating negatively charged components on the painful area of the body surface over which the device is applied and attract positively charged components, as a result of which a beneficial effect on pain will be accrued.

A pain scale measures a patient's pain intensity or other features. Pain scales are based on self-report, observational (behavioral), or physiological data. Self-report is considered primary and should be obtained if possible (since pain is a quale by definition, and therefore assessment based on any set scale of expected outcomes from similar cases can fail to provide useful clinical data). Wong-Baker FACES pain rating scale, for example, shows a series of faces ranging from a happy face at 0, "No hurt" to a crying face at 10 "Hurts worst".

A set of three different devices are useful for pain relief by reducing Wong-Baker FACES pain rating scale in at least one unit (n=23). In a first device, a negative static electrical field strength of 8.5 kilo Volts per inch of 8.5 kilo Volts (KV) per inch may be applied to the polymer sheet adapted as a barrier in a second system, a negative static electrical field strength of 8.5 kilo Volts per inch of 8.5 KV per inch that is gradually released from a polymer sheet to a Human's skin; and in a third system, a static electricity device that generates about from about 2.5 to about 15 kilo Volts per inch according to a predefined protocol (see e.g., Example 5).

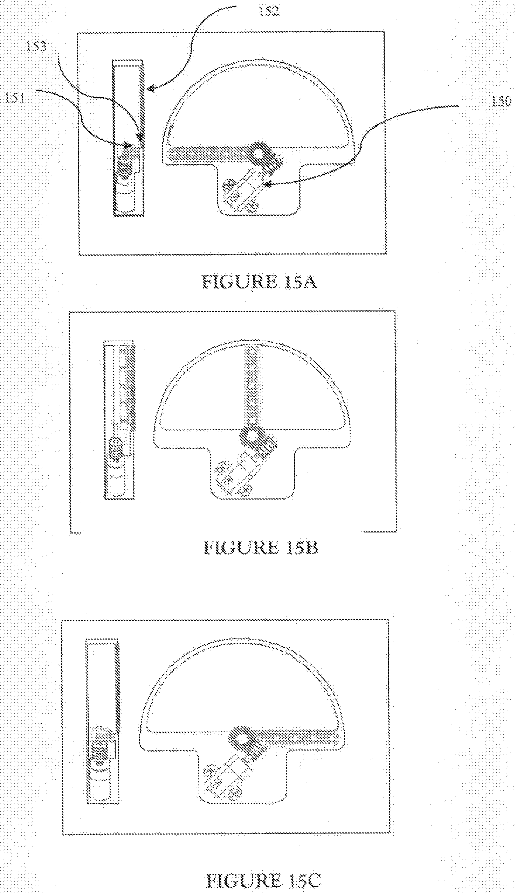

Reference is now made to Figures 15-17 which illustrate the electrostatic generator of the present invention comprising a motor gear, a sheet assembly and a brush having variety of movements.

Figure 15A-C illustrates a semicircle motion brush 151, brush lifter 153, sheet assembly 152 and motor gear 150 thereby, the brush is swung back and forth in a circular bidirectional motion similar to windscreen wiper movement and rubbing the sheet assembly 152 to generate static electricity.

Figure 15A illustrates the non-activated configuration, figure 15B illustrates the charging configuration when brush is rubbing the sheet assembly and figure 15C illustrates brush position when is detached from the sheet.

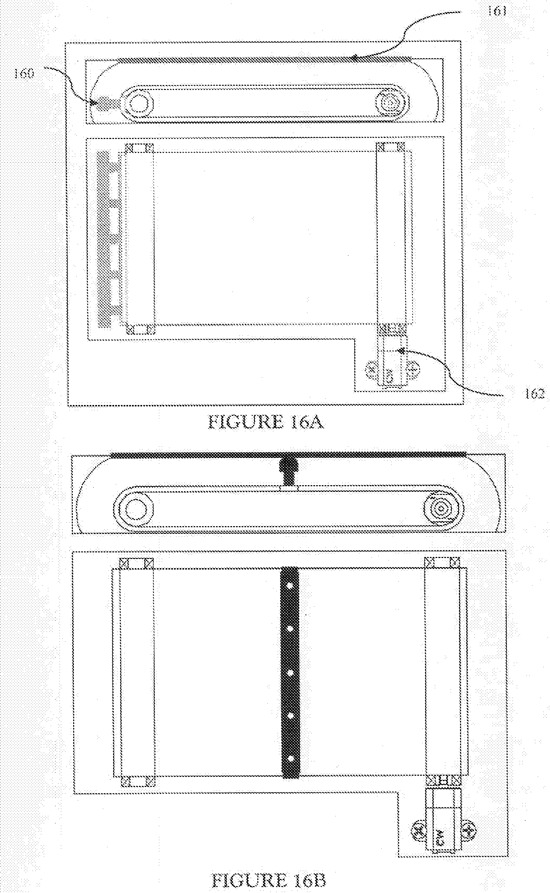

Figure 16A-C illustrates a Linear motion brush 160 sheet assembly 161 and motor gear 162 thereby, the brush attached to a continuous loop conveyor belt and moving along the conveyor belt in a circular movement back and forth or in a unidirectional motion, rubbing the polymer sheet assembly to generate static electricity. Furthermore, the brush is pivoting about one end and reverse itself.

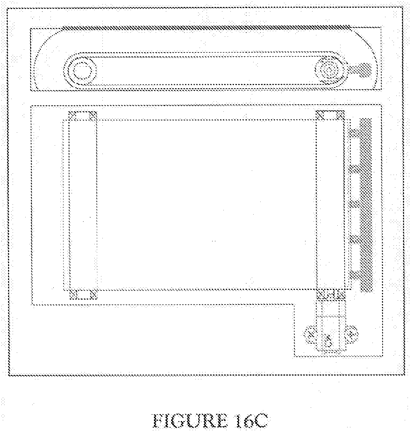

Figure 16A illustrates the non-activated configuration, figure 16B illustrates the charging configuration when the brush is rubbing the sheet assembly and figure 16C illustrates the brush position when is detached from the sheet.

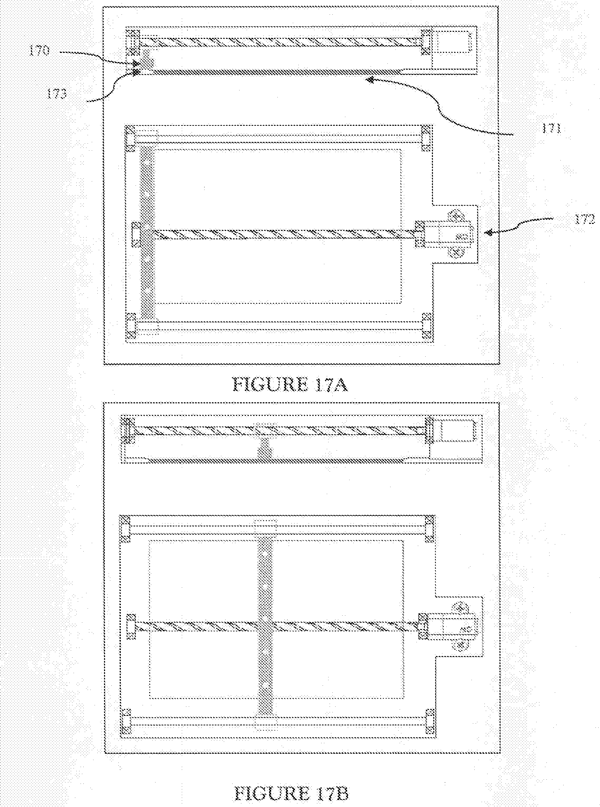

Figure 17A-C illustrates a Lead screw motion brush 170, brush lifter 173, sheet assembly 171 and motor gear 172 thereby, the brush is moving in a linear bidirectional motion back and forth rubbing the polymer sheet assembly to generate static electricity.

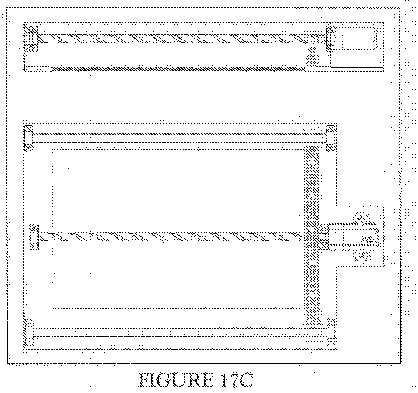

Figure 17A illustrates the non-activated configuration, figure 17B illustrates the charging configuration when brush is rubbing the sheet assembly and figure 17C illustrates the brush position when it is not in contact with the sheet.

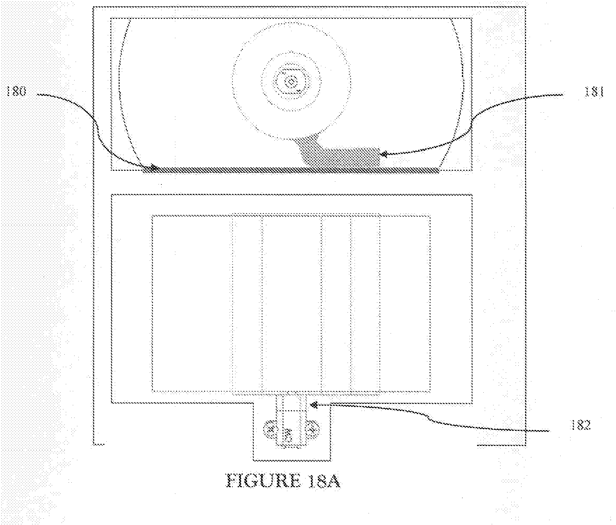

Figure 18A-C illustrates a Turning motion brush 181, sheet assembly 180 and motor gear 182 thereby, the brush is a flexible brush moving in a unidirectional motion. Furthermore, the brush pivots at one end at the same direction or back and forth.

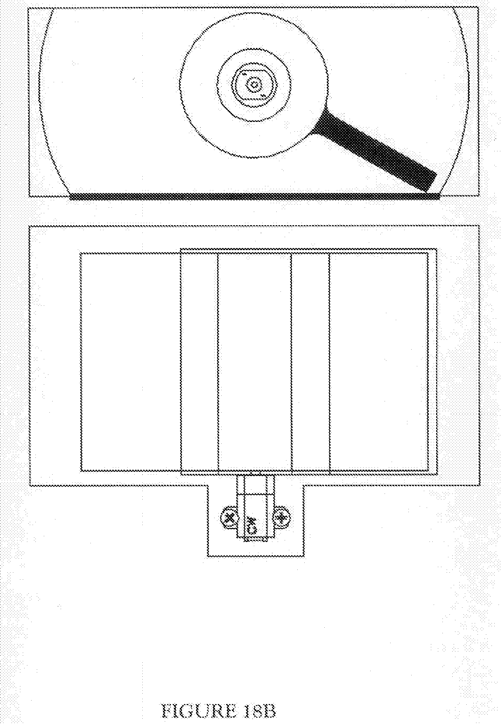

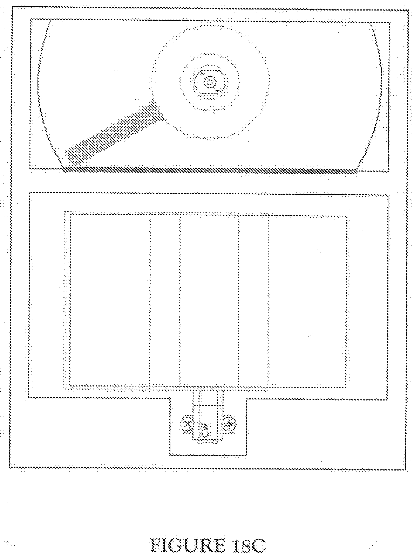

Figure 18A illustrates the non-activated configuration, figure 18B illustrates the charging configuration when the flexible brush is rubbing the sheet assembly and figure 18C illustrates the brush position when it is not in contact with the sheet assembly. Without forming part of the invention, the disclosure provides a method of generating an electro-static field and a method for elevating pain by synergistically alleviating pain in at least one portion of patient's body.

The method comprising steps of :(*a*) providing a portable handheld static-electricity generator comprising a movable brush, a polymer sheet assembly comprising a rubbing-surface upon which the brush rubs, an automatically-operated mechanical unit and a polymer sheet assembly, (*b*) actuating the brush in respect to the polymer sheet thereby, rubbing the polymer assembly by means of the brush and,
(c) generating an electro-static field; and (d) attaching the sheet to the portion of patient's body.

The exemplary method may further comprise the step of providing the brush with Activated Configuration in which moving brush is rubbing the sheet so as static electricity is generated; and a Non-Activated Configuration in which the brush is detached from the sheet and static electricity is not generated.

The method may further comprise the step of providing the brush with a brush activating mechanism which switch the brush from the Non-Activated Configuration to the Activated Configuration and *vice versa.*

The step of actuating the brush may be in a linear motion, semicircle motion, lead screw motion or turning motion.

The static electricity may be activated along a predefined period t ranging from one or more ranges selected from a group consisting of 1 to 45-120 seconds; 1 to 12-44 seconds. 0.5 to 1-11 seconds, 1-3 to 6-12 seconds, 4-6 to 8-12 seconds and any combination thereof. Furthermore, t provides a train of n pulses of static-electricity, n is integer equal or greater 2.

At least two pulses from the n pulses of static-electricity may be of equal duration.

At least two pulses from the n pulses of static-electricity may be of different duration.

## Claims

1. A portable handheld static-electricity generator (10) for alleviating pain in at least one portion of patient's body, comprising
a) a movable brush assembly (60),
b) an automatically-operated mechanical unit (50);
wherein additionally comprising a polymer assembly (40) comprising a rubbing-surface portion (42) upon which said movable brush assembly (60) is configured to rub; said polymer assembly having affinity to a defined charge such that when said movable brush assembly (60) rubs said rubbing surface (42) of said polymer assembly (40) for a predefined time period, a static electricity is generated .

2. The static-electricity generator (10) of claim 1, wherein said defined charged is positive charge or negative charge.

3. The static-electricity generator (10) of one of claims 1 and 2, wherein when said polymer assembly (40) with affinity to negative charge, and said brush assembly (60) is with affinity to positive charge.

4. The static-electricity generator (10) of any one of claims 1 to 3, wherein said mechanical unit (50) comprises a DC motor (56) and gear-head thereof, provided in connection with a spur gear (57) by means of a worm gear (55a, 55b).

5. The static-electricity generator (10) of any one of claims 1 to 4, wherein said brush assembly (60) comprises a brushing material and a brush body; said brushing material being affixed to said brush body.

6. The static-electricity generator (10) of claim 4, wherein said brush assembly (60) is at least reversibly affixed on said spur gear.

7. The static-electricity generator (10) of any one of claims 1 to 6, wherein said polymer assembly (40) comprises said rubbing-surface portion as an upper portion upon which the static electricity is generated and a lowest surface portion attached to said patient's body, through which the charge is discharged.

8. The static-electricity generator (10) of claim 4, wherein said polymer assembly (40) is adjacent to said brush assembly (60) such that by operating said motor (56), said spur gear (57) activates said brush assembly (60) which is continuously rubbing said rubbing-surface portion of said polymer assembly (40) for a predefined time, thereby generating static electricity upon said polymer assembly by attaching said charged assembly to said patient's body for thereby alleviating pain.

9. The static-electricity generator (10) of any one of claims 1 to 8, wherein said brush assembly (60) is activated in a linear motion, semicircle motion, lead screw motion or turning motion.

10. The static-electricity generator (10) of any one of claims 1 to 9, wherein said polymer assembly (40) is composed of a material selected from the group consisting of silicone rubber, silicon, Teflon, PVC, polyethylene ,rubber, Butyl rubber, Hypalon rubber, Santoprene rubber, Epichlorohydrin rubber, Latex rubber, PET, Polyimide, Polystyrene, Cellulose nitrate and any combination thereof.

11. The static-electricity generator (10) of any one of claims 1 to 10, wherein said brush assembly (60) is composed of a material selected from the group consisting of Polyurethane foam, hair, nylon, Nylatron, glass, paper cotton and any combination thereof.

12. The static-electricity generator (10) of any one of claims 1 to 11, wherein said predefined time period (*t*) ranges from one or more ranges selected from a group consisting of 1 to 45-120 seconds; 1 to 12-44 seconds. 0.5 to 1-11 seconds, 1-3 to 6-12 seconds, 4-6 to 8-12 seconds and any combination thereof.

## Patentansprüche

1. Ein tragbarer, in der Hand zu haltender Elektrostatikgenerator (10) zur Linderung von Schmerzen in mindestens einem Teil des Körpers eines Patienten, umfassend:
a) eine bewegbare Bürstenbaugruppe (60),
b) eine automatisch betriebene mechanische Einheit (50);
wobei er zusätzlich eine Polymerbaugruppe (40) umfasst, die einen Reiboberflächenabschnitt (42) umfasst, auf dem die bewegbare Bürstenbaugruppe (60) zu reiben vorgesehen ist;
wobei die Polymerbaugruppe eine Affinität für eine definierte Ladung hat, so dass dann, wenn die bewegbare Bürstenbaugruppe (60) über eine vorgegebene Zeitspanne an der Reiboberfläche (42) der Polymerbaugruppe (40) reibt, eine statische Elektrizität erzeugt wird.

2. Der Elektrostatikgenerator (10) nach Anspruch 1, wobei die definierte Ladung eine positive Ladung oder eine negative Ladung ist.

3. Der Elektrostatikgenerator (10) nach einem der Ansprüche 1 und 2, wobei wenn die Polymerbaugruppe (40) eine Affinität für eine negative Ladung hat und
die Bürstenbaugruppe (60) eine Affinität für eine positive Ladung hat.

4. Der Elektrostatikgenerator (10) nach einem der Ansprüche 1 bis 3, wobei die mechanische Einheit (50) einen Gleichstrommotor (56) und einen Getriebekopf dazu umfasst, der mittels eines Schneckenrads (55a, 55b) in Verbindung mit einem Stirnrad (57) bereitgestellt ist.

5. Der Elektrostatikgenerator (10) nach einem der Ansprüche 1 bis 4, wobei die Bürstenbaugruppe (60) ein Bürstenmaterial und einen Bürstenkörper umfasst; wobei das Bürstenmaterial an dem Bürstenkörper befestigt ist.

6. Der Elektrostatikgenerator (10) nach Anspruch 4, wobei die Bürstenbaugruppe (60) zumindest reversibel an dem Stirnrad befestigt ist.

7. Der Elektrostatikgenerator (10) nach einem der Ansprüche 1 bis 6, wobei die Polymerbaugruppe (40) umfasst: den Reiboberflächenabschnitt als oberen Abschnitt, auf dem die statische Elektrizität erzeugt wird, und einen untersten Oberflächenabschnitt, der an dem Körper des Patienten angebracht wird, durch den die Ladung abgegeben wird.

8. Der Elektrostatikgenerator (10) nach Anspruch 4, wobei die Polymerbaugruppe (40) an die Bürstenbaugruppe (60) angrenzt, so dass durch eine Betätigung des Motors (56) das Stirnrad (57) die Bürstenbaugruppe (60) aktiviert, die für eine vordefinierte Zeit kontinuierlich über den Reiboberflächenabschnitt der Polymerbaugruppe (40) reibt, wodurch auf der Polymerbaugruppe eine statische Elektrizität erzeugt wird, durch Anbringen der geladenen Baugruppe am Körper des Patienten, um dadurch Schmerzen zu lindern.

9. Der Elektrostatikgenerator (10) nach einem der Ansprüche 1 bis 8, wobei die Bürstenbaugruppe (60) in einer linearen Bewegung, einer Halbkreisbewegung, einer Führungsschraubbewegung oder einer Drehbewegung aktiviert wird.

10. Der Elektrostatikgenerator (10) nach einem der Ansprüche 1 bis 9, wobei die Polymerbaugruppe (40) aus einem Material gebildet ist, das ausgewählt ist aus der Gruppe bestehend aus Silikongummi, Silikon, Teflon, PVC, Polyethylen ,gummi, Butylgummi, Hypalongummi, Santoprengummi, Epichlorhydringummi, Latexgummi, PET, Polyimid, Polystyrol, Cellulosenitrat und irgendeiner Kombination davon.

11. Der Elektrostatikgenerator (10) nach einem der Ansprüche 1 bis 10, wobei die Bürstenbaugruppe (60) von einem Material gebildet wird, das ausgewählt ist aus der Gruppe bestehend aus Polyurethanschaum, Haaren, Nylon, Nylatron, Glas, Papier Baumwolle und irgendeiner Kombination davon.

12. Der Elektrostatikgenerator (10) nach einem der Ansprüche 1 bis 11, wobei die vorgegebene Zeitspanne (t) im Bereich von einem oder mehreren Bereichen liegt, die ausgewählt sind aus der Gruppe bestehend aus 1 bis 45-120 Sekunden; 1 bis 12-44 Sekunden, 0,5 bis 1-11 Sekunden, 1-3 bis 6-12 Sekunden, 4-6 bis 8-12 Sekunden und irgendeiner Kombination davon.

## Revendications

1. Un générateur portable d'électricité statique (10) du type tenu en main destiné à soulager la douleur dans au moins une partie du corps d'un patient, qui comprend :
a) un assemblage de balais mobiles (60) ;
b) une unité mécanique (50) du type qui peut être activée de manière automatique ;
dans lequel le générateur en question comprend en outre un assemblage polymère (40) qui comprend une portion faisant office de surface de frottement (42) sur laquelle est configuré ledit assemblage de balais mobiles (60) pour exercer un frottement ; ledit assemblage polymère possédant une affinité pour une charge qui a été définie, d'une manière telle que, lorsque ledit assemblage de balais mobiles (60) exerce un frottement contre ladite surface de frottement (42) dudit assemblage polymère (40) pendant un laps de temps qui a été prédéfini, une électricité statique est générée.

2. Le générateur d'électricité statique (10) conformément à la revendication 1, dans lequel ladite charge qui a été définie représente une charge positive ou une charge négative.

3. Le générateur d'électricité statique (10) conformément à l'une quelconque des revendications 1 et 2, dans lequel, lorsque ledit assemblage polymère (40) manifeste une affinité pour une charge négative, ledit assemblage de balais (60) manifeste une affinité pour une charge positive.

4. Le générateur d'électricité statique (10) conformément à l'une quelconque des revendications 1 à 3, dans lequel ladite unité mécanique (50) comprend un moteur du type à courant continu (56) et une tête d'engrenage de ce dernier, prévue en liaison avec un engrenage de type cylindrique (57) au moyen d'un engrenage à vis sans fin (55a, 55b).

5. Le générateur d'électricité statique (10) conformément à l'une quelconque des revendications 1 à 4, dans lequel ledit assemblage de balais (60) comprend une matière de brossage et un corps de balai ; ladite matière de brossage étant fixée audit corps de balai.

6. Le générateur d'électricité statique (10) conformément à la revendication 4, dans lequel ledit assemblage de balais (60) est au moins fixé de manière réversible audit engrenage de type cylindrique.

7. Le générateur d'électricité statique (10) conformément à l'une quelconque des revendications 1 à 6, dans lequel ledit assemblage polymère (40) comprend ladite portion faisant office de surface de frottement sous la forme d'une portion supérieure sur laquelle est générée l'électricité statique et une portion faisant office de surface la plus inférieure qui est fixée audit corps du patient, par l'intermédiaire de laquelle la charge est déchargée.

8. Le générateur d'électricité statique (10) conformément à la revendication 4, dans lequel ledit assemblage polymère est disposé en position adjacente audit assemblage de balais (60) d'une manière telle que, lors de l'activation du moteur (56) en question, ledit engrenage de type cylindrique (57) active ledit assemblage de balais (60) qui exerce un frottement en continu sur ladite portion faisant office de surface de frottement, dudit assemblage polymère (40) pendant un laps de temps qui a été prédéfini, dans le but de générer de cette manière une électricité statique sur ledit assemblage polymère par l'intermédiaire de la fixation dudit assemblage à l'état chargé audit corps du patient pour ainsi soulager la douleur de ce dernier.

9. Le générateur d'électricité statique (10) conformément à l'une quelconque des revendications 1 à 8, dans lequel ledit assemblage de balais (60) est activé sous la forme d'un mouvement linéaire, d'un mouvement semi-circulaire, d'un mouvement à la manière d'une vis sans fin ou d'un mouvement de rotation.

10. Le générateur d'électricité statique (10) conformément à l'une quelconque des revendications 1 à 9, dans lequel ledit assemblage polymère (40) se compose d'une matière qui est choisie parmi le groupe constitué par du caoutchouc de silicone, un polymère à base de silicium, du téflon, du PVC, du caoutchouc de polyéthylène, du caoutchouc butyle, du caoutchouc Hypalon®, du caoutchouc Santoprène®, du caoutchouc d'épichlorhydrine, du caoutchouc latex, du PET, du polyimide, du polystyrène, du nitrate de cellulose, ainsi que l'une quelconque de leurs combinaisons.

11. Le générateur d'électricité statique (10) conformément à l'une quelconque des revendications 1 à 10, dans lequel ledit assemblage de balais (60) se compose d'une matière qui est choisie parmi le groupe constitué par de la mousse de polyuréthane, une matière à base de poils, du nylon, du Nylatron®, du verre, du papier, du coton, ainsi que l'une quelconque de leurs combinaisons,

12. Le générateur d'électricité statique (10) conformément à l'une quelconque des revendications 1 à 11, dans lequel ledit laps de temps (*t*) qui a été prédéfini se situe dans une ou plusieurs plages qui est/sont choisie(s) parmi le groupe constitué par : une plage de 1 à 45-120 secondes ; une plage de 1 à 12-44 secondes ; une plage de 0,5 à 1-11 secondes ; une plage de 1-3 à 6-12 secondes ; une plage de 4-6 à 8-12 secondes ; ainsi que l'une quelconque de leurs combinaisons.
